(19) Europäisches Patentamt
European Patent Office
Office européen des brevets

(11) Publication number: 0 114 675
A2

# (12) EUROPEAN PATENT APPLICATION

(21) Application number: 84100559.8

(22) Date of filing: 19.01.84

(51) Int. Cl.³: G 03 C 7/32
G 03 C 7/26, C 07 D 249/18
C 07 D 405/04

(30) Priority: 19.01.83 JP 7150/83

(43) Date of publication of application: 01.08.84 Bulletin 84/31

(84) Designated Contracting States: DE GB

(71) Applicant: FUJI PHOTO FILM CO., LTD.
210 Nakanuma Minami Ashigara-shi
Kanagawa 250-01(JP)

(72) Inventor: Sakanoue, Kei
c/o FUJI PHOTO FILM CO. LTD. 210 Nakanuma
Minami Ashigara-shi Kanagawa(JP)

(72) Inventor: Ichijima, Seiji
c/o FUJI PHOTO FILM CO. LTD. 210 Nakanuma
Minami Ashigara-shi Kanagawa(JP)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22(DE)

## (54) Color photographic silver halide light-sensitive material.

(57) A color photographic silver halide light-sensitive material is described. The material is characterized by containing a development inhibitor-releasing coupler capable of releasing an inhibitor having a degree of diffusion ranging between 0.4 and 0.95 on reacting with an oxidation product of a developing agent during development. This light-sensitive material provides images which are greatly improved in sharpness without causing other problems.

Fig. 1

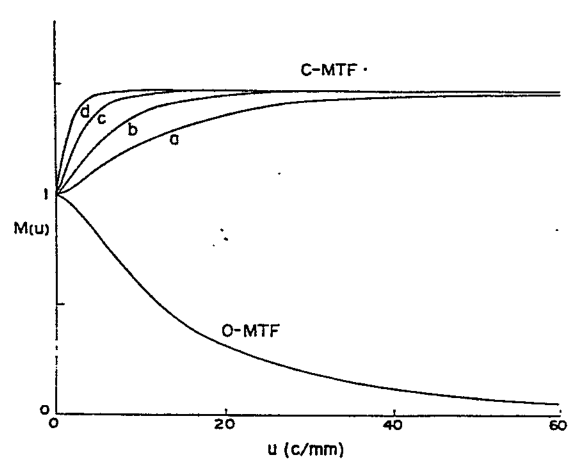


EP 0 114 675 A2

GRÜNECKER · DR. KINKELDEY · DR. STOCKMAIR
DR. SCHUMANN · JAKOB · DR. BEZOLD · MEISTER
HILGERS · DR. MEYER-PLATH
MAXIMILIANSTR. 58 · 8000 MÜNCHEN 22

# COLOR PHOTOGRAPHIC SILVER HALIDE LIGHT-SENSITIVE MATERIAL

## FIELD OF THE INVENTION

The present invention relates to a color photographic silver halide light-sensitive material and, more particularly, to a color photographic silver halide light-sensitive material for cameras which is improved in sharpness.

## BACKGROUND OF THE INVENTION

With a recent spread of Size 110 cameras, it is desired that a smaller image plane, when enlarged, produces images of similar quality to those from a large image plane. For this purpose it is essential to provide a color photographic silver halide light-sensitive material which has such superior granularity and sharpness that even if the magnification of enlargement is increased, the quality of the resulting print is not substantially degraded.

Various methods of increasing the sharpness of images are known. It is also well known that increasing the edge effect and decreasing light-scattering in photographic light-sensitive material are effective measures for increasing sharpness. The former method of increasing the edge effect utilizes a concentration gradient of an inhibiting substance during development.

Typical examples of such inhibiting substances include iodide and bromide ions which are released when silver halide is developed in a developer, and oxidation products of developing agents. In addition, the compounds as described in, for example, U.S. Patents 3,227,554, 3,701,783, 3,615,506 and 3,617,291 are known to increase the edge effect positively. The DIR compounds and DIR couplers, as described in Japanese Patent Publication No. 34933/80, are known as the most improved of the foregoing compounds. Even with these DIR couplers, however, it is not possible to increase the edge effect beyond a certain limit. The reason is that addition of large amounts of DIR couplers to enhance the edge effect releases large amounts of inhibiting substances, inevitably resulting in a reduction in the maximum concentration and also a reduction in sensitivity.

To overcome the problem as described above, it is necessary to increase the amount of, for example, silver being coated. This will increase light-scattering due to silver halide and conversely lower an MTF curve (in connection with the MTF curve, see Mees, The Theory of Photographic Process, 3rd Ed., Macmillan Co.), degrading the sharpness of not only a DIR coupler-added layer, but also its underlying layer. In addition, this increase in the amount of silver coated undesirably increases production costs.

The present invention is intended to develop novel DIR couplers which eliminate the above-described disadvantages of conventional DIR couplers and which are capable of greatly increasing the edge effect while maintaining the degree of inhibition (degree of reduction in the maximum concentration) within a given range, and to provide a color photographic light-sensitive material having improved sharpness in a low frequency region, as prepared using the novel DIR couplers.

## SUMMARY OF THE INVENTION

As a result of extensive investigations, it has been found that there is a certain relation between diffusibility of a development-inhibiting substance and an MTF curve, and that a development inhibitor-releasing coupler capable of releasing an inhibitor having a degree of diffusion ranging between 0.4 and 0.95 upon reaction with an oxidation product of a developing agent in the stage of development is useful for the purpose.

Therefore, the present invention provides a color photographic silver halide light-sensitive material characterized by containing a development inhibitor-releasing coupler capable of releasing an inhibitor having a degree of diffusion ranging between 0.4 and 0.95 on reacting with an oxidation product of a developing agent in the stage of development.

## BRIEF DESCRIPTION OF THE DRAWINGS

Figure 1 shows C-MTF curves and an O-MTF curve as obtained when the degree of diffusion is changed as shown below while maintaining the degree of inhibition at the same level:

a .... 0.1, b .... 0.2, c .... 0.4, d .... 0.8;

and

Figure 2 shows a theoretical MTF curve as derived from the curves of Figure 1.

## DETAILED DESCRIPTION OF THE INVENTION

Simulation of the MTF curve when the diffusibility of the inhibiting substance is changed was performed as follows.

A practical negative light-sensitive material was exposed to soft X-rays, developed, and measured by means of a micro densitometer to determine the shape of an edge formed. Various parameters for a diffusion equation were determined so that the shape of the edge could be reproduced by simulation. From this diffusion equation it is possible to determine a concentration distribution of an inhibiting substance. Further, from the thus-obtained equation for the concentration distribution of an inhibiting substance, a chemical fog function was determined, and this function was transformed into a Fourier series whereby C-MTF (a) as shown in

Figure 1 was obtained. When the diffusion constant of the development-inhibiting substance was raised from (a) to (d), there were obtained curves C-MTF (a) to C-MTF (d) in which no light-scattering occurred.

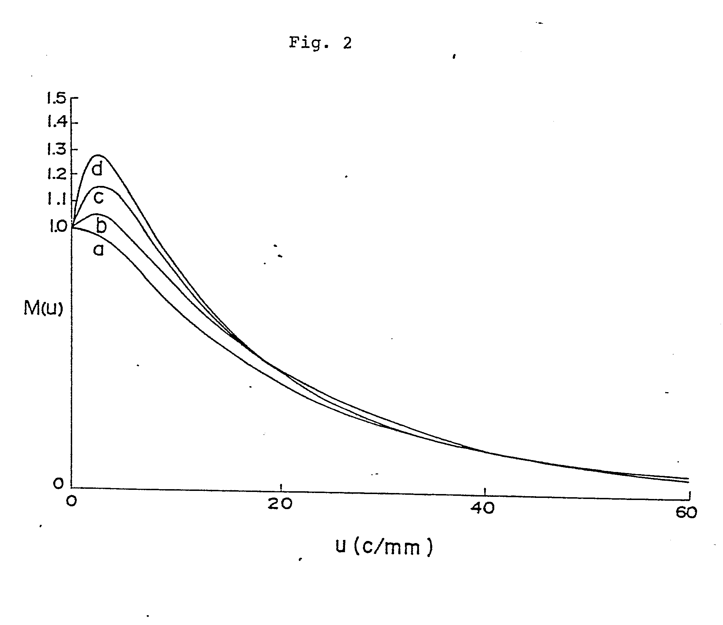

As diffusibility is high, the C-MTF value in a low space frequency region is high. Curve O-MTF indicates an MTF curve in which the edge effect cannot be obtained at all, i.e., a certain amount of light-scattering occurs. Since the practical MTF value is the product of an MTF value, MC(u), on the C-MTF curve and MO(u) on the O-MTF curve, the theoretical MTF value when only the diffusibility of the development-inhibiting substance is changed can be represented by Figure 2. From Figure 2 it can be seen that if the diffusibility of a releasing group of a DIR coupler is increased, the edge effect is increased and, in particular, MTF in a low space frequency region increases greatly.

The degree of diffusion of a development-inhibiting substance as used herein can be determined by the following method.

A multilayer color light-sensitive material is prepared by providing the layers as described hereinafter on a transparent support. This material is designated as Sample H.

<u>First Layer</u>: Red-Sensitive Silver Halide Emulsion Layer

A silver iodobromide emulsion (silver iodide: 5 mol%; mean size: 0.4 μ) is provided with sensitivity by adding Sensitizing Dye I as used in Example 1 in an amount of $6\times10^{-5}$ mol per mol of silver. A gelatin coating solution containing the above-sensitized emulsion and Coupler C-2 in an amount of 0.0015 mol per mol of silver is coated so that the amount of silver coated is 1.8 $g/m^2$ (film thickness: 2 μ), whereby the red-sensitive silver halide emulsion is prepared.

<u>Coupler C-2</u>

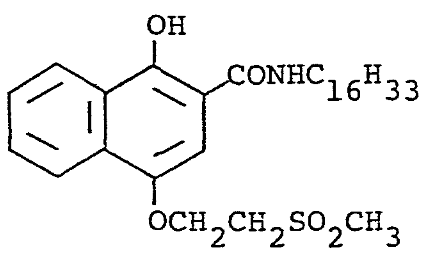

<u>Second Layer</u>: Gelatin Layer

Containing polymethyl methacrylate particles (diameter: about 1.5 μ) of the silver iodobromide emulsion (not having red-sensitivity) as used in the preparation of First Layer (amount of silver coated: 2 $g/m^2$; film thickness: 1.5 μ).

Each layer contains a gelatin hardener and a surfactant as well as the above-described ingredients.

A color light-sensitive material is prepared which has the same structure as Sample H except that the silver iodobromide emulsion of Second Layer is not contained. This material is designated as Sample G.

Samples G and H were each exposed to light through a wedge and, thereafter, processed in the same manner as in Example 1 except that the developing time is changed to 130 seconds. A development inhibitor is added to the developer till the density of Sample G drops to 1/2 of the original value. The degree of reduction in the density of Sample H at this time is used as a measure of diffusibility of the development inhibitor in the silver halide emulsion layer. The results are shown in Table 1.

As can be seen from Table 1, the higher the degree of diffusion, the larger the amount of the inhibitor added must be in order to provide the same degree of inhibition. That is, it can be concluded that the diffusibility of a development inhibitor in a silver halide film is inversely proportional to its development-inhibiting ability. Hence, when a development inhibitor of high diffusibility is used, the amount of the inhibitor being added must be increased since its degree of inhibition is low. In practice, however, if the degree of diffusion of the development inhibitor exceeds

0.95, the use of a DIR coupler containing the development inhibitor as a releasing group gives rise to the problem that the amount of the DIR coupler being added becomes very large, leading to an increase in the thickness of the emulsion film and a degradation of a high frequency component of MTF.

As apparent from the Examples described hereinafter, it is only development inhibitors having a degree of diffusion ranging between 0.4 and 0.95 that can be recognized to increase the edge effect and substantially improve MTF. For example, in the case of a benzotriazole compound, if the degree of diffusion changes from 0.3 to 0.7, the MTF value increases from 1.05 to 1.21 (see Samples 101 and 103 as described hereinafter). Also with a mercaptotetrazole compound, if the degree of diffusion changes from 0.2 to 0.44, the MTF value increases from 1.03 to 1.10 (see Samples 102 and 104 as described hereinafter). Thus, it can be understood that increasing the degree of diffusion is effective for increasing the edge effect.

In summary, the higher the degree of diffusion of a DIR compound, the greater the edge effect of the DIR compound. However, the development-inhibiting ability of the DIR compound decreases in inverse proportion to the degree of diffusion thereof and thus it is

necessary to increase the amount of the DIR compound being added in order to obtain the same development-inhibiting ability.

TABLE 1

Degrees of Diffusion of Development Inhibitors

| Development Inhibitor | Amount (M) | Rate of Reduction of Density Sample A (%) | Rate of Reduction of Density Sample B (%) | Degree of Diffusion (B/A) |
|---|---|---|---|---|
| 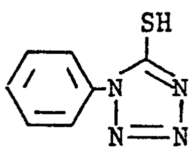 | $0.75\times10^{-4}$ | 50 | 10 | 0.20 |
| 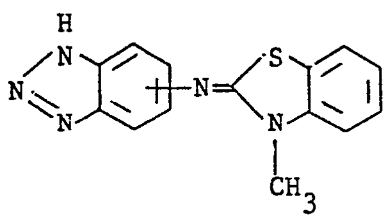 | $0.5\times10^{-4}$ | 50 | 15 | 0.30 |
| 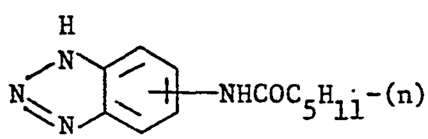 | $2.5\times10^{-4}$ | 50 | 42 | 0.84 |
| 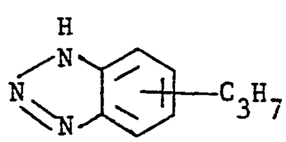 | $1.5\times10^{-4}$ | 55 | 34 | 0.62 |

(cont'd)

| Development Inhibitor | Amount (M) | Rate of Reduction of Density Sample A (%) | Rate of Reduction of Density Sample B (%) | Degree of Diffusion (B/A) |
|---|---|---|---|---|
| 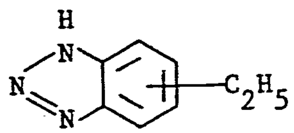 | $2\times10^{-4}$ | 52 | 36 | 0.69 |
| 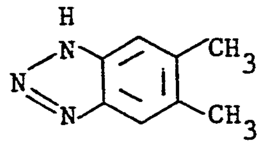 | $2\times10^{-4}$ | 50 | 35 | 0.70 |
| 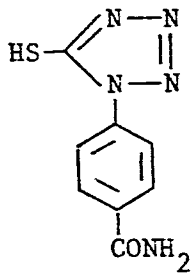 | $2.0\times10^{-4}$ | 48 | 21 | 0.44 |
| 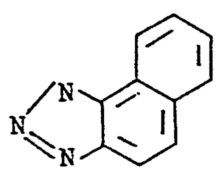 | $3.0\times10^{-4}$ | 47 | 41 | 0.88 |

From the results of Table 1, it can be seen that benzotriazole-based development inhibitors are advantageously used since they have high diffusibility and greatly increase the edge effect, i.e., the MTF value.

It has been found that the development inhibitors having the structures as described hereinafter have particularly high diffusibility and are particularly preferred for use in the present invention.

That is, couplers containing the groups represented by the general formulae (I), (II) and (III) as releasing groups and couplers containing a naphthotriazolyl group (e.g., a 1H-naphtho[1,2-d]triazolyl group) as a releasing group are preferably used in the present invention.

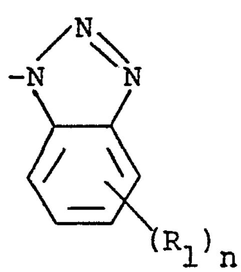

(I)

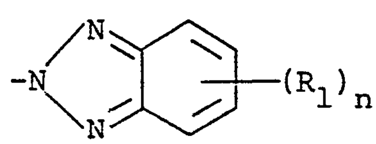

(II)

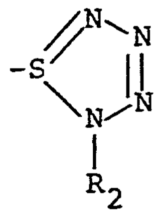

(III)

wherein n is 1 or 2; when n is 1, $R_1$ is a pentanamido group, a hexanamido group, a propoxy group, an N-butanecarbamoyl group, an N-pentylcarbamoyl group, an N-hexylcarbamoyl group, a butoxy group, a pentyloxy group, or an ethyl group, and when n is 2, $R_1$ is a methyl group; and $R_2$ is a propyl group or a substituted phenyl group (the substituent is selected from the group consisting of an N-methylsulfamoyl group, a ureido group, a carbamoyl group, an N-methylcarbamoyl group, a methanesulfonamido or acetamido group, and an alkoxyacylamino group; and when the substituent contains an alkyl group, the alkyl group preferably contains 1 or 2 carbon atoms).

Of the DIR couplers containing the above-described releasing groups of high diffusibility, the compounds represented by the general formulae (IV), (V) and (VI) as shown below are preferred.

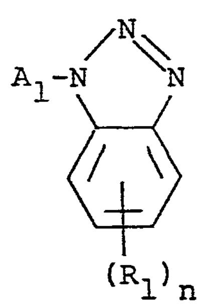

(IV)

or

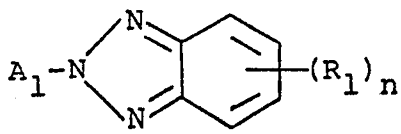

(IV)

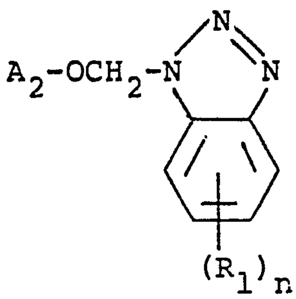

(V)

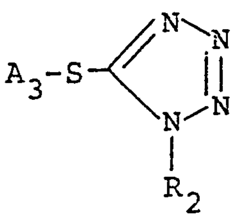

(VI)

wherein $A_1$ is a radical derived from a yellow coupler or a magenta coupler by removing one hydrogen atom positioned at the coupling position thereof; $A_2$ is a radical derived from a cyan coupler by removing one hydrogen atom positioned at the coupling position thereof; $A_3$ is a radical derived from a yellow coupler, a magenta coupler or a cyan coupler by removing one hydrogen atom from the coupling position thereof; and $R_1$, $R_2$ and n are as defined above.

Of the compounds represented by the general formulae (IV) and (VI) in which $A_1$ and $A_3$ are each a radical derived from a yellow coupler by removing one hydrogen atom positioned at the coupling position thereof, the compounds containing $A_1$ or $A_3$ each represented by the general formulae (VII) and (VIII) are more preferred.

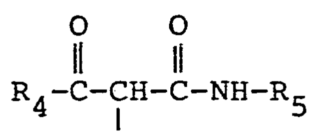

(VII)

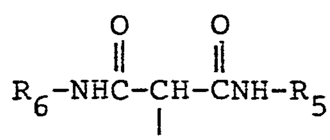

(VIII)

wherein $R_4$ is an aliphatic or aromatic group, and $R_5$ and $R_6$ are each an aromatic group.

When in the general formulae (IV) and (VI) $A_1$ and $A_3$ each represent a radical derived from a magenta coupler by removing one hydrogen atom located at the coupling position thereof, the compounds containing $A_1$ or $A_3$ each represented by the general formula (IX) are preferred.

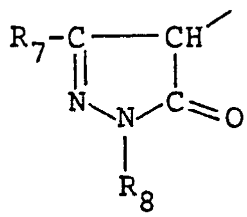

(IX)

wherein $R_7$ is an aliphatic group, an anilino group, an alkylamino group, a dialkylamino group, a cyclic amino group, or an acylamino group, and $R_8$ is an aliphatic group or an aromatic group.

When in the general formulae (V) and (VI) $A_2$ and $A_3$ are each a radical derived from a cyan coupler by removing one hydrogen atom located at the coupling position thereof, the compounds containing $A_2$ or $A_3$ each represented by the general formula (X) are preferred.

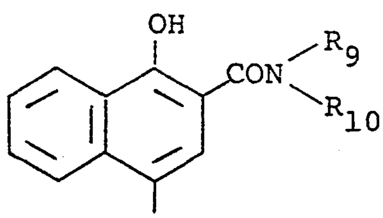

(X)

wherein $R_9$ and $R_{10}$ are each a hydrogen atom, an aliphatic group, or an aromatic group.

As for the general formulae (IV) and (VI), $A_1$ and $A_3$ are each a radical derived from a colorless coupler by removing one hydrogen atom located at the the

coupling position thereof, those coupler nuclei described in, for example, U.S. Patents 4,052,213, 4,088,491, 3,632,345, 3,958,993 and 3,961,959 can be used.

When $R_4$, $R_7$, $R_8$, $R_9$ or $R_{10}$ represents an aliphatic group, it preferably contains from 1 to 22 carbon atoms, and may be substituted or unsubstituted, chain-like or cyclic, and saturated or unsaturated. Examples of preferred substituents include an alkoxy group, an aryloxy group, an acylamino group, a halogen atom, and an alkylthio group, which may be further substituted. Examples of useful aliphatic groups include a methyl group, a tert-butyl group, an isoamyl group, a butyl group, a hexyl group, a dodecyl group, a hexadecyl group, a cyclohexyl group, a 3-(2,4-di-tert-amylphenoxy)propyl group, a 3-dodecyloxypropyl group, and an α-(succinimido)isopropyl group.

When $R_4$, $R_5$, $R_6$, $R_8$, $R_9$ or $R_{10}$ represents an aromatic group, it may be substituted. Examples of substituents include a halogen atom (e.g., a chlorine atom and a fluorine atom), an alkoxy group containing 32 or less carbon atoms, an alkyl group, an alkoxycarbonyl group, an alkoxycarbonylamino group, an aliphatic amido group, an alkylsulfamoyl group, an alkanesulfone group, a nitro group, a hydroxy group, a cyano group, and a ureido group. If the substituent contains an alkyl moiety, the alkyl moiety may be further substituted.

Examples of useful aromatic groups include a 2-chlorophenyl group, a 2-methoxyphenyl group, a 4-[2-(2,4-di-tert-amylphenoxy)butanamido]phenyl group, a 2-chloro-5-[2-(2,4-di-tert-amylphenoxy)butanamido]phenyl group, a 2-chloro-5-tetradecyloxycarbonylphenyl group, a 2-tetradecyloxyphenyl group, a 2,4,6-trichlorophenyl group, a 4-methoxyphenyl group, a phenyl group and a 2,5-dichlorophenyl group.

When $R_7$ represents an anilino group, it may be substituted. Examples of substituents include a halogen atom, a hydroxy group, a cyano group, an alkoxy group, an alkyl group, an alkoxycarbonyl group, an aliphatic amido group, an aromatic amido group, an alkylsulfamoyl group, and an alkanesulfonamido group. If these substituents contain an alkyl or phenyl moiety, it may be further substituted. Examples of useful anilino groups include an anilino group, a 4-methoxyanilino group, a 2-chloro-5-tetradecanamidoanilino group, a 2-methoxy-5-tetradecyloxyanilino group and a 2-chloro-5-octadecenylsuccinimidoanilino group.

When $R_7$ represents an alkylamino group or a dialkylamino group, each alkyl group may be chain-like or cyclic, substituted or unsubstituted, and saturated or unsaturated. The number of carbon atoms is preferably 32 or less. Examples of substituents include an alkyl

group, an aryloxy group, an aryl group and a hydroxy group. Examples of preferred alkylamino groups or diàlkylamino groups include a dibutylamino group, a bis-(2-hydroxyethyl)amino group, a dibenzylamino group, an octadecylamino group and a 3-(2,4-di-tert-amylphenoxy)-propylamino group.

The cyclic amino group represented by $R_7$ is preferably 5-membered or 6-membered. Examples of useful cyclic amino groups include a pyrrolidinyl group, a piperazinyl group and a morpholinyl group.

The acylamino group represented by $R_7$ may be either an aliphatic amido group or an aromatic amido group. The aliphatic amido group preferably contains from 1 to 22 carbon atoms and may be substituted or unsubstituted, chain-like or cyclic, and saturated or unsaturated. Examples of preferred substituents include an alkoxy group, an aryloxy group, an acylamino group, a halogen atom and an anilino group. Examples of useful aliphatic amido groups include a tetradecanamido group, a tert-butyramido group, an acetamido group, a 3-(2,4-di-tert-acylphenoxy)butyramido group, and a 2-(3-pentadecyl-phenoxy)butyramido group. In the aromatic amido group, the aromatic group (in particular, a phenyl group) may be substituted. Examples of substituents include a halogen atom, an acylamino group, an alkoxy group, an

alkyl group and a hydroxy group. Examples of useful aromatic amido groups include a benzamido group, a 4-methoxybenzamido group, a 5-[2-(2,4-di-tert-amylphenoxy)-butyramido]benzamido group and a 4-methoxy-5-[2-(2,4-di-tert-amylphenoxy)butyramido]benzamido group.

The compounds as described above can be easily prepared by, for example, the methods described in U.S. Patents 4,234,678, 3,227,554, 3,617,291, 3,958,993, 4,149,886, 3,933,500, Japanese Patent Application (OPI) Nos. 56837/82, 13239/76 (the term "OPI" as used herein refers to a "published unexamined Japanese patent application"), British Patents 2,072,363, 2,070,266, and Research Disclosure, No. 21228, Dec., 1981.

Some preparation examples are given below.

PREPARATION EXAMPLE 1

Synthesis of Compound (1)

2-Pivaloyl-2'-chloro-5'-[4-(2,4-di-tert-amylphenoxy)butyramido]acetanilide (23.5 g) was mixed with 100 mℓ of chloroform, and 6.6 g of bromine was added dropwise thereto. After washing with water, the resulting solution was added dropwise at room temperature to a solution of 12.1 g of 5,6-dimethylbenzotriazole and 8.3 g of triethylamine in 50 mℓ of N,N-dimethylformamide. The resulting mixture was stirred at room temperature for 2 hours and then washed with 500 mℓ of water. After

washing with diluted hydrochloric acid while continuing the separation of oil layers, washing with water was repeated till the solution became neutral. After the separation of oil layers, the solution was concentrated under reduced pressure, and the residue thus obtained was recrystallized from hexane and isopropanol to form 16 g of the desired compound, Coupler (1), m.p. 141-142°C.

PREPARATION EXAMPLE 2

Synthesis of Compound (2)

3-{3-[2-(2,4-di-tert-Amylphenoxy)butyramido]-benzamido}-1-(2,4,6-trichlorophenyl)-2-pyrazoline-5-one (25 g) was dissolved in chloroform, and 5.4 g of bromine was added dropwise thereto. The resulting solution was palced in a separatory funnel and washed with water, and then the solvent was distilled off. The residue thus obtained was mixed with 15.7 g of 5,6-dimethylbenzotriazole and 15 mℓ of sulfolane, and stirred at 100°C for 10 hours. The mixture was cooled to room temperature and, after addition of 200 mℓ of chloroform, washed with water. The oil layer was concentrated under reduced pressure, and the residue thus obtained was subjected to column chromatography using silica gel as a filter. After eluting with ethyl acetate, fractions containing the desired product were collected and concentrated to form 11 g of the desired compound.

## PREPARATION EXAMPLE 3

Synthesis of Compound (3)

First Step: Preparation of 4-(5,6-Dimethylbenzotriazol-1-yl)methoxy-2-naphthoic Acid

A methanol solution (50 mℓ) containing 20.4 g of 4-hydroxy-2-naphthoic acid and 12 g of sodium methoxide was mixed with 200 mℓ of N,N-dimethylformamide, and a solution of 20 g of 1-chloromethyl-5,6-dimethylbenzotriazole in 50 mℓ of acetonitrile was added dropwise thereto at 40°C. They were reacted for 1 hour, and poured into an aqueous solution of hydrochloric acid. Crystals precipitated were collected by filtration to obtain 22 g of the desired compound.

Second Step: Preparation of 4-(5,6-dimethylbenzotriazol-1-yl)methoxy-2-naphthoic Acid Phenyl Ester

A mixture of 10.9 g of 4-(5,6-dimethylbenzotriazol-1-yl)methoxy-2-naphthoic acid as prepared above and 3.4 g of phenol was mixed with 120 mℓ of acetonitrile, and 5.4 g of thionyl chloride was added thereto. The resulting mixture was heated under reflux for 3 hours. The solvent was distilled off under reduced pressure, and the residue was recrystallized from acetonitrile to form 11 g of the desired compound.

Third Step: Preparation of Compound (3)

A mixture of 11 g of 4-(5,6-dimethylbenzotriazol-1-yl)methoxy-2-naphthoic acid phenyl ester as

prepared in Second Step and 6.6 g of 3-(2,4-di-tert-amylphenoxy)propylamine was dissolved in 50 mℓ of tetrahydrofuran, and the mixture was heated under reflux for 8 hours. The solvent was distilled off under reduced pressure, and the residue was recrystallized from ethyl acetate to obtain 5 g of the desired compound, m.p., 184-185°C.

PREPARATION EXAMPLE 4

Synthesis of Compound (6)

N,N'-Bis(2-chloro-5-dodecyloxycarbonylphenyl)-malondiamide (22.4 g) was mixed with 100 mℓ of chloroform, and 4.8 g of bromine was added dropwise thereto at room temperature. The reaction mixture was washed with water and then added dropwise to a solution of 13.9 g of 5-hexanamidobenzotriazole and 3 g of triethylamine in 50 mℓ of N,N-dimethylformamide. They were reacted at room temperature for 3 hours, and then transferred to a separatory funnel and washed with water. After further washing with diluted hydrochloric acid, washing with water was repeated till the solution became neutral. The oil layer was separated and then concentrated under reduced pressure. The residue thus obtained was recrystallized from ethyl acetate and acetonitrile to form 14 g of the desired compound, m.p., 150-153°C.

Typical examples of the couplers of the invention are given below.

(1)

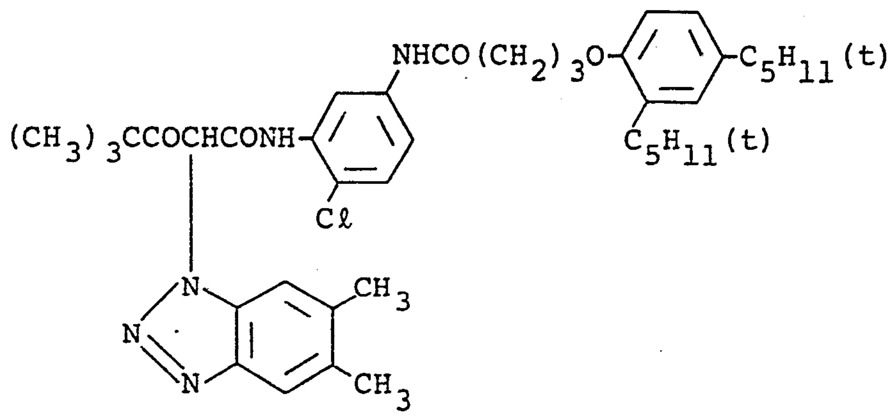

(2)

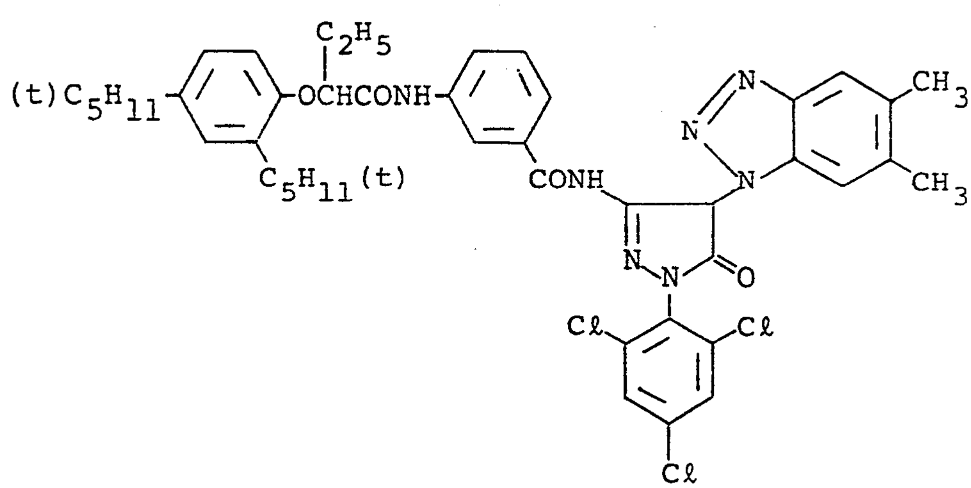

(3)

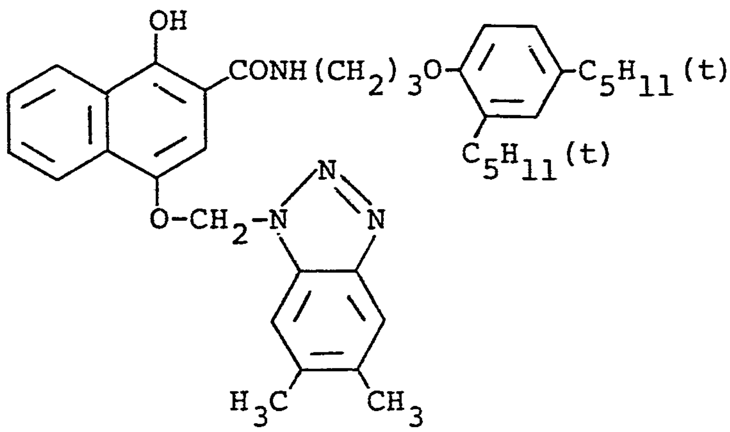

(4)

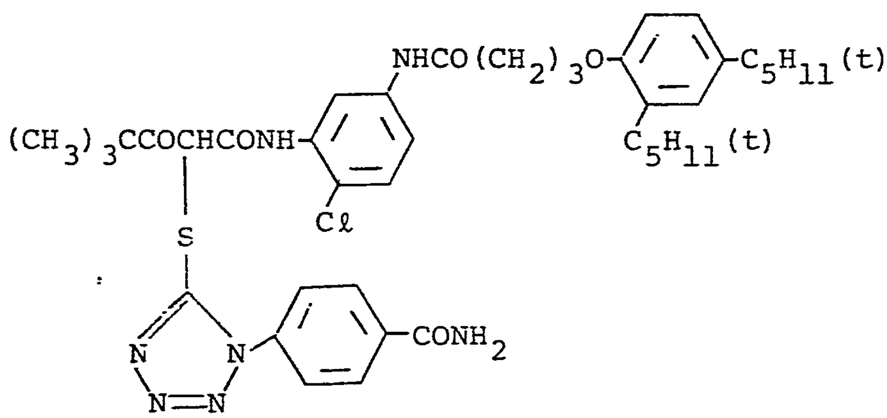

(5)

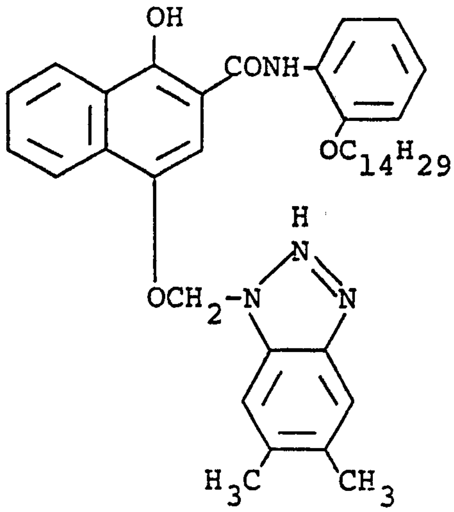

(6)

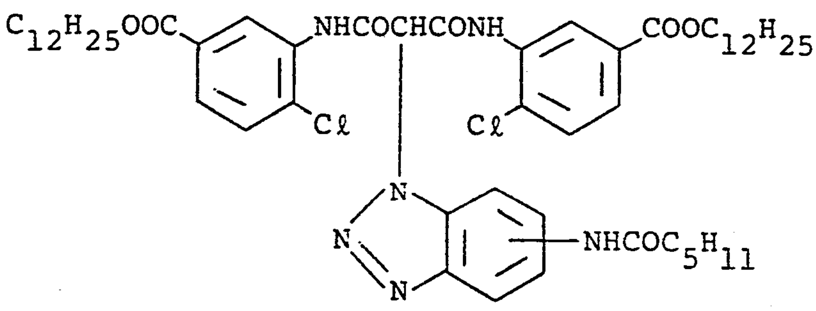

(7)

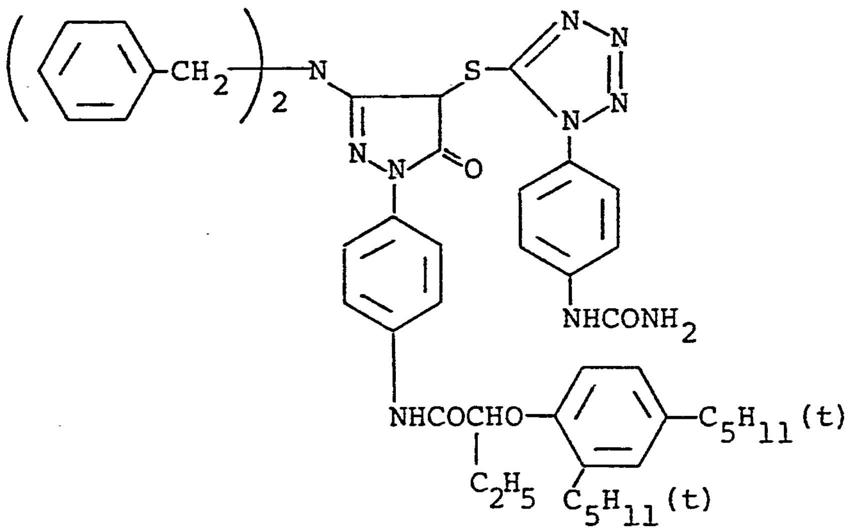

(8)

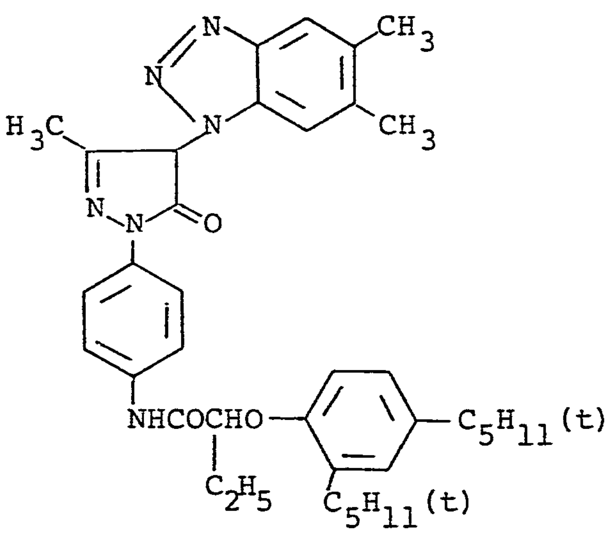

(9)

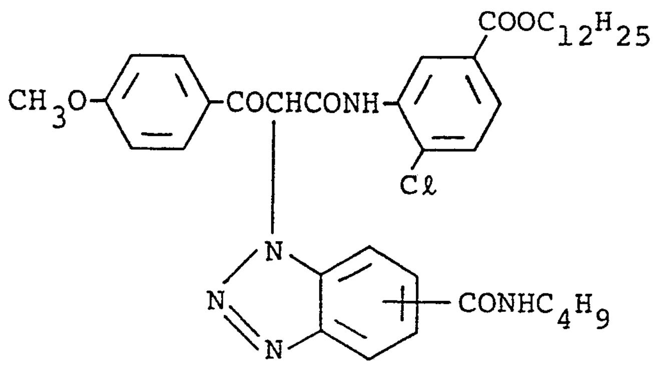

(10)

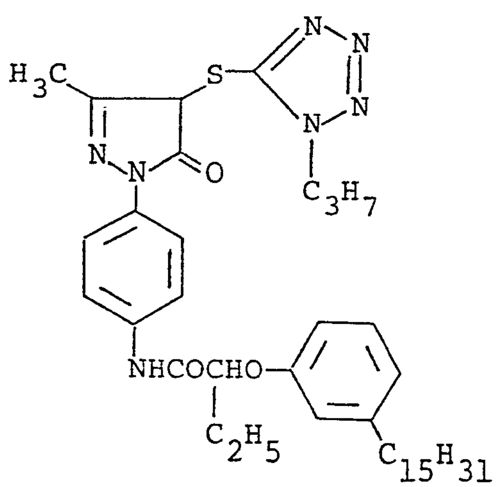

(11)

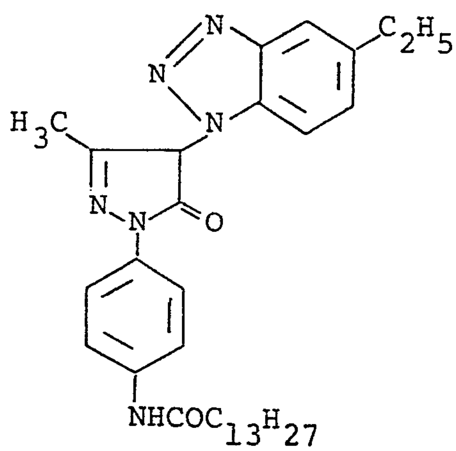

(12)

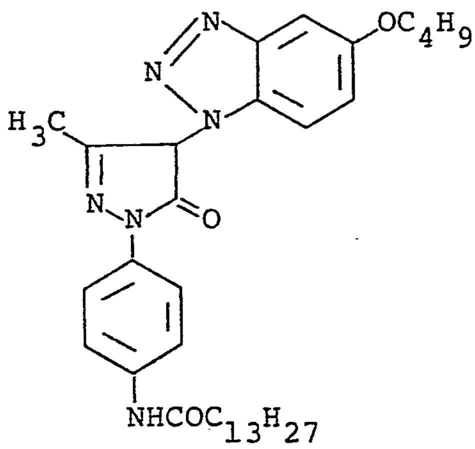

(13)

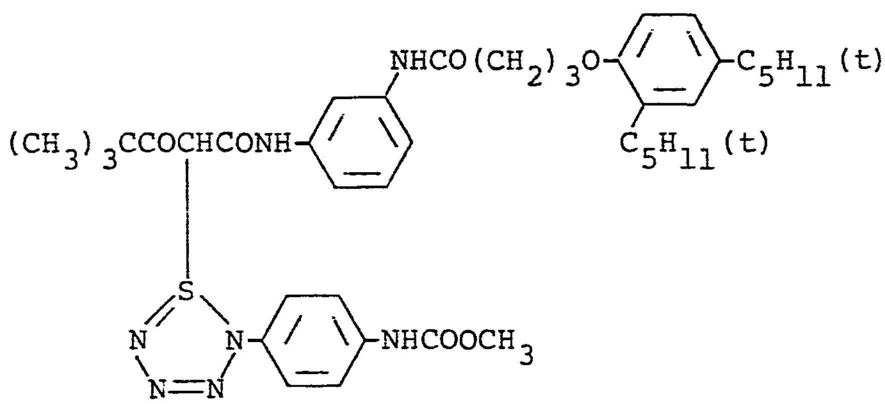

The DIR coupler of the invention is added in an amount of from 0.00001 to 0.5 mol, preferably from 0.001 to 0.05 mol, per mol of silver. This DIR coupler may be added to an emulsion layer, or intermediate layers such as an intermediate layer sandwiched between a high sensitive emulsion layer and a low sensitive emulsion layer having the same color sensitivity.

The DIR coupler of the invention can be introduced in a silver halide emulsion layer by known techniques such as the method as described in U.S. Patent 2,322,027. For example, the coupler is dissolved in alkyl phthalates (e.g., dibutyl phthalate and dioctyl phthalate), phosphates (e.g., diphenyl phosphate, triphenyl phosphate, tricresyl phosphate, and dioctylbutyl phosphate), citrates (e.g., tributyl acetylcitrate), benzoates (e.g., octyl benzoate), alkylamides (e.g., diethyllaurylamide), fatty acid esters (e.g., dibutoxyethyl succinate and dioctyl acetate), trimesicates (e.g., tributyl trimesicate), or organic solvents having a boiling point ranging between about 30 and 150°C, for example, lower alkyl acetates such as ethyl acetate and butyl acetate, sec-butyl alcohol, ethyl propionate, methyl isobutyl ketone, β-ethoxyethyl acetate, and methyl Cellosolve acetate and, thereafter, dispersed in hydrophilic colloid. The above-described high boiling and low boiling organic solvents may be used in combination with each other. In addition, it is possible to use the dispersion method using polymers as described in Japanese Patent Publication No. 39853/76 and Japanese Patent Application (OPI) No. 59943/76.

If the DIR coupler contains an acid group, such as carboxylic acid or sulfonic acid, it is introduced in the hydrophilic colloid as an alkaline aqueous solution.

Gelatin is used advantageously as a binder or protective colloid for photographic emulsions. Other hydrophilic colloids can be used. For example, gelatin derivatives, graft polymers of gelatin with other polymers, proteins such as albumin and casein, cellulose derivatives such as hydroxyethyl cellulose, carboxymethyl cellulose and cellulose sulfate, sugar derivatives such as sodium alginate and starch derivatives, and various synthetic hydrophilic polymeric substances, homopolymers or copolymers, such as polyvinyl alcohol, polyvinyl alcohol hemiacetal, poly(N-vinyl)pyrrolidone, polyacrylic acid, polymethacrylic acid, polyacrylamide, polyvinylimidazole and polyvinylpyrazole can be used.

It is possible to use lime-treated gelatin, acid-treated gelatin and enzyme-treated gelatin as described in Bull. Soc. Sci. Phot., Japan, No. 16, page 30 (1966). In addition, hydrolyzates and enzyme-decomposition products of gelatin can be used.

Examples of gelatin derivatives which can be used include compounds prepared by reacting gelatin with various compounds such as acid halides, acid anhydrides, isocyanates, bromoacetic acid, alkanesultones, vinylsulfonamides, maleinimide compounds, polyalkylene oxides and epoxy compounds. Typical examples are shown in, for example, U.S. Patents 2,614,928, 3,132,945, 3,186,846,

3,312,553, British Patents 861,414, 1,033,139, 1,005,784 and Japanese Patent Publication No. 26845/67.

Graft polymers of gelatin which can be used include compounds prepared by grafting acrylic acid, methacrylic acid and their derivatives such as esters and amides, and homo- or copolymers of vinyl-based monomers, such as acrylonitrile and styrene, onto gelatin. In particular, graft polymers of gelatin and polymers having a certain extent of compatibility with gelatin, such as polymers of acrylic acid, methacrylic acid, acrylamide, and hydroxyalkyl methacrylate, are preferred. These compounds are described in, for example, U.S. Patents 2,763,625, 2,831,767 and 2,956,884.

Typical synthetic hydrophilic polymeric substances are the compounds described in, for example, West German Patent Application (OLS) No. 2,312,708, U.S. Patents 3,620,751, 3,879,205 and Japanese Patent Publication No. 7561/68.

In the photographic emulsion layer of the photographic light-sensitive material of the invention, the silver halide may be any of silver bromide, silver iodobromide, silver iodochlorobromide, silver chlorobromide and silver chloride. A preferred example is silver iodobromide containing 15 mol% or less of silver iodide. Particularly preferred is silver iodobromide containing from 2 to 12 mol% of silver iodide.

Although the mean grain size of silver halide grains in the photographic emulsion is not critical, it is preferably not greater than 3 μ. The mean grain size is determined based on projected areas with a grain diameter as the grain size in the case of spherical or nearly spherical grains, or an edge length as the grain size in the case of cubic grains.

The grain size distribution may be narrow or broad.

Silver halide grains in the photographic emulsion may be in a regular crystal form, e.g., cubic or octahedral, or in an irregular crystal form, e.g., spherical or plate-like, or in a composite crystal form thereof. Furthermore, they may be a mixture of grains having various crystal forms.

Silver halide grains may be composed of an inner portion and a skin layer, having different phases, or may be composed of a uniform phase. Furthermore, they may be grains in which a latent image is formed mainly on the surface thereof, or grains in which a latent image is formed mainly in the interior thereof.

Photographic emulsions as used herein can be prepared by the methods as described in, for example, P. Glafkides, Chimie et Physique Photographique, Paul Montel (1967), G.F. Duffin, Photographic Emulsion Chemistry, The Focal Press Co. (1966), and V.L. Zelikman

et al., Making and Coating Photographic Emulsions, The Focal Press Co. (1964). That is, any of an acid process, a neutral process, an ammonia process, and so forth can be employed, and soluble silver salts and soluble halides can be reacted in any suitable manner, such as by a single jet process, a double jet process, and a combination thereof.

A method, so-called reverse mixing method, in which grains are formed in the presence of excess of silver ions can be employed. As one embodiment of the double jet process, a so-called controlled double jet process in which the pAg of a liquid layer where silver halide is formed is kept constant can also be employed. This method produces a silver halide emulsion in which the crystal form is regular and the grain size is nearly uniform.

Two or more silver halide emulsions which are prepared separately can be used in admixture with each other.

The formation or physical ripening of silver halide grains may be performed in the presence of cadmium salts, zinc salts, lead salts, thallium salts, iridium salts or its complex salts, rhodium salts or its complex salts, iron salts or its complex salts, and so forth.

For the removal of soluble salts from the emulsion after the formation of precipitate or physical ripening, a noodle water-washing method in which gelatin is gelled may be used, or a flocculation method utilizing inorganic salts, anionic surfactants, anionic polymers (e.g., polystyrenesulfonic acid), or gelatin derivatives (e.g., acylated gelatin and carbamoylated gelatin) may be used.

The silver halide emulsion is usually subjected to chemical sensitization. For this chemical sensitization, for example, the methods as described in H. Frieser ed., Die Grundlagen der Photographischen Prozesse mit Silberhalogeniden, Akademische Verlagsgesellschaft (1968), pages 675-734 can be employed.

That is, a sulfur sensitization method using sulfur-containing compounds capable of reacting with activated gelatin and silver (e.g., thiosulfates, thioureas, mercapto compounds and rhodanines), a reduction sensitization method using reducible substances (e.g., stannous salts, amines, hydrazine derivatives, formamidinesulfinic acid, and silane compounds), a noble metal sensitization method using noble metal compounds (e.g., complex salts of gold, and the metals of Group VIII of the Periodic Table, such as platinum, iridium and palladium), and so forth can be used singly or in combination with each other.

The sulfur sensitization method is described in detail in, for example, U.S. Patents 1,574,944, 2,410,689, 2,278,947, 2,728,668 and 3,656,955; the reduction sensitization method, in, for example, U.S. Patents 2,983,609, 2,419,974 and 4,054,458; and the noble metal sensitization method, in, for example, U.S. Patents 2,399,083, 2,448,060, and British Patent 618,061.

Various compounds can be incorporated in the photographic emulsion as used herein for the purpose of preventing fog during the process of production, storage or photographic processing of light-sensitive material, or of stabilizing its photographic performance. For this purpose can be used a number of compounds known as antifoggants or stabilizers, such as azoles, e.g., benzothiazolium salts, nitroindazoles, triazoles, benzotriazoles, and benzimidazoles (particularly nitro or halogen-substituted products); heterocyclic mercapto compounds, e.g., mercaptothiazoles, mercaptobenzothiazoles, mercaptobenzimidazoles, mercaptothiadiazoles, mercaptotetrazoles (particularly 1-phenyl-5-mercaptotetrazole), and mercaptopyrimidines; the above-described heterocyclic mercapto compounds further containing a water-soluble group such as a carboxyl group or a sulfone group; thioketo compounds, e.g., oxazolinethione; azaindenes, e.g., tetraazaindenes (particularly 4-hydroxy-

substituted (1,3,3a,7)tetraazaindenes); benzenethiosulfonic acids; and benzenesulfinic acid.

For more detailed examples and methods of using them, U.S. Patents 3,954,474, 3,982,947, 4,021,248 and Japanese Patent Publication No. 28660/77 can be referred to.

The photographic emulsion layer or other hydrophilic colloid layers of the light-sensitive material of the invention may contain therein various surfactants as coating aids or for various purposes of, e.g., preventing charging, improving sliding properties and emulsification dispersion, preventing adhesion or improving photographic characteristics (such as acceleration of development, high contrast and sensitization).

For example, nonionic surfactants such as saponin (steroid-base), alkylene oxide derivatives (e.g., polyethylene glycol, a polyethylene glycol/polypropylene glycol mixture, polyethylene glycol alkyl ethers or polyethylene glycol alkylaryl ethers, polyethylene glycol esters, polyalkylene glycol sorbitan esters, polyalkylene glycol alkylamines or amides, and a silicone/polyethylene oxide adduct), glycidol derivatives (e.g., alkenylsuccinic acid polyglyceride and alkylphenol polyglyceride), fatty acid esters of polyhydric alcohols, and alkyl esters of saccharides;

anionic surfactants containing acid groups, such as a carboxyl group, a sulfo group, a phospho group, a sulfate group, and a phosphate group, e.g., alkyl carboxylates, alkyl sulfonates, alkylbenzenesulfonates, alkylnaphthalenesulfonates, alkylsulfates, alkyl phosphates, N-acyl-N-alkyltaurines, sulfosuccinates, sulfoalkylpolyoxyethylene alkylphenyl ethers, and polyoxyethylene alkyl phosphates; amphoteric surfactants such as amino acids, aminoalkylsulfonic acids, alkylbetaines, and amine oxides; and cationic surfactants such as aliphatic or aromatic quaternary ammonium salts, heterocyclic quaternary ammonium salts (e.g., pyridinium and imidazolium), and aliphatic or heterocyclic phosphonium or sulfonium salts can be used.

The photographic emulsion layer of the photographic light-sensitive material of the invention may contain polyalkylene oxide or its derivatives, such as ethers, esters and amines, thioether compounds, thiomorpholines, quaternary ammonium salt compounds, urethane derivatives, urea derivatives, imidazoles, 3-pyrazolidones, and so forth for the purpose of increasing sensitivity or contrast, or accelerating development. For example, the compounds as described in U.S. Patents 2,400,532, 2,423,549, 2,716,062, 3,617,280, 3,772,021, 3,808,003 and British Patent 1,488,991 can be used.

The photographic light-sensitive material of the invention may contain dispersions of water-insoluble or water-sparingly-soluble synthetic polymers in the photographic emulsion layer or other hydrophilic colloid layers thereof for the purpose of, e.g., improving dimensional stability. Examples of such synthetic polymers include homo- or copolymers of monomers such as alkyl acrylate or methacrylate, alkoxyalkyl acrylate or methacrylate, glycidyl acrylate or methacrylate, acrylamide or methacrylamide, vinyl esters (e.g., vinyl acetate), acrylonitrile, olefins, and styrene, and copolymers of the foregoing monomers and monomers such as acrylic acid, methacrylic acid, α,β-unsaturated dicarboxylic acid, hydroxyalkyl acrylate or methacrylate, sulfoalkyl acrylate or methacrylate, and styrenesulfonic acid. For example, the compounds described in U.S. Patents 2,376,005, 2,739,137, 2,853,457, 3,062,674, 3,488,708, 3,525,620, 3,607,290, 3,635,715, 3,645,740, British Patents 1,186,699 and 1,307,373 can be used.

In photographic processing of the light-sensitive material of the invention, any of the known methods and known processing solutions as described in, for example, Research Disclosure, No. 176, pages 28-30 (RD-17643) can be used. Alternatively, if desired, a color photographic processing to form a color image may

be applied. The processing temperature is usually chosen within the range of from 18 to 50°C, although lower temperatures than 18°C or higher temperatures than 50°C may be employed.

As a special developing system, there may be employed a method in which a developing agent is incorporated in a light-sensitive material, for example, in its emulsion layer, and the light-sensitive material is developed by treating it in an alkaline aqueous solution. Of these developing agents, hydrophobic ones can be incorporated in the emulsion layer by various techniques such as the methods as described in Research Disclosure, No. 169 (RD-16928), U.S. Patent 2,739,890, British Patent 813,253, and West German Patent 1,547,763. Such a developing treatment may be performed in combination with a silver salt-stabilizing treatment using thiocyanates.

Fixers having the formulations that are commonly used can be used in the present invention. As fixing agents, as well as thiosulfates and thiocyanates, organosulfur compounds which are known to have a fixing effect can be used. These fixers may contain a water-soluble aluminum salt as a hardener.

Dye images can be formed by the usual method such as a negative-positive process (which is described

in, for example, Journal of the Society of Motion Picture and Television Engineers, Vol. 61 (1953), pages 667-701).

A color developer is generally an alkaline aqueous solution containing a color developing agent. As color developing agents, the known primary aromatic amine developing agents, such as phenylenediamines (e.g., 4-amino-N,N'-diethylaniline, 3-methyl-4-amino-N',N-diethylaniline, 4-amino-N-ethyl-N-β-hydroxyethylaniline, 3-methyl-4-amino-N-ethyl-N-β-hydroxyethylaniline, 3-methyl-4-amino-N-ethyl-N-β-methanesulfonamidoethylaniline, and 4-amino-3-methyl-N-ethyl-N-β-methoxyethylaniline), can be used. In addition, the compounds as described in L.F.A. Mason, Photographic Processing Chemistry, Focal Press Co. (1966), pages 226-229, U.S. Patents 2,193,015, 2,592,364 and Japanese Patent Application (OPI) No. 64933/73 may be used.

The color developer may further contain pH buffers, development inhibitors, antifoggants, and so forth. In addition, if necessary, hard water-softening agents, preservatives, organic solvents, development accelerators, dye-forming couplers, competitive couplers, foggants, auxiliary developing agents, tackifiers, polycarboxylic acid-based chelating agents, and antioxidants may be incorporated.

Typical examples of such additives are described in, for example, Research Disclosure, (RD-17643), U.S. Patent 4,083,723, and West German Patent Application (OLS) No. 2,622,950.

After color development, the photographic emulsion layer is usually bleached. This bleaching treatment may be performed separately from or simultaneously with the fixing treatment. Bleaching agents which can be used include the compounds of polyvalent metals such as iron (III), cobalt (III), chromium (IV) and copper (II), peracids, quinones, and nitroso compounds.

For example, ferricyanides; dichromates; organic complex salts of iron (III) or cobalt (III), such as complex salts of organic acids such as aminopolycarboxylic acids (e.g., ethylenediaminetetraacetic acid, nitrilotriacetic acid, and 1,3-diamino-2-propanoltetraacetic acid), citric acid, tartaric acid, and malic acid; persulfates; permanganates; and nitrosophenol can be used. Of these compounds, potassium ferricyanide, sodium iron (III) ethylenediaminetetraacetate, and ammonium iron (III) ethylenediaminetetraacetate are especially useful. The ethylenediaminetetraacetic acid iron (III) complex salt is useful in both the independent bleaching solution and the combined bleaching and fixing solution.

To the bleach or bleach-fixing solution can be added various additives as well as the bleach accelerators described in, for example, U.S. Patents 3,042,520, 3,241,966, Japanese Patent Publication Nos. 8506/70 and 8836/70, and the thiol compounds described in Japanese Patent Application (OPI) No. 65732/78.

The photographic emulsion as used herein may be subjected to spectral sensitization using methine dyes, etc.

Useful sensitizing dyes are the compounds as described in, for example, German Patent 929,080, U.S. Patents 2,493,748, 2,503,776, 2,519,001, 2,912,329, 3,656,959, 3,672,897, 4,025,349, British Patent 1,242,588 and Japanese Patent Publication No. 14030/69.

These sensitizing dyes may be used in the usual manner, but may be used in combination with each other. Combinations of sensitizing dyes are often used particularly for the purpose of color sensitization. Typical examples are described in U.S. Patents 2,688,545, 2,977,229, 3,397,060, 3,522,052, 3,527,641, 3,617,293, 3,628,964, 3,666,480, 3,672,898, 3,679,428, 3,814,609, 4,026,707, British Patent 1,344,281, Japanese Patent Publication Nos. 4936/68, 12375/78, Japanese Patent Application (OPI) Nos. 110618/77 and 109925/77.

The present invention includes a multilayer multicolor photographic light-sensitive material comprising a support and at least two layers having different spectral sensitivities. This multilayer natural color photographic light-sensitive material usually bears at least one red-sensitive emulsion layer, at least one multicolor sensitive emulsion layer, and at least one blue-sensitive emulsion layer on the support. The order of these layers is not critical, but can be determined appropriately.

It is usual for the red-sensitive emulsion layer to contain a cyan-forming coupler, for the green-sensitive emulsion layer to contain a magenta-forming coupler, and for the blue-sensitive emulsion layer to contain a yellow-forming coupler. In some cases, however, different combinations are employed.

An exposure time ranging between 1/1,000 and 1 second which is usually used for cameras can be, of course, employed in the present invention. In addition, an exposure of shorter than 1/1,000 second, and an exposure of from $1/10^4$ to $1/10^6$ second using a xenon flash lamp or a cathode ray tube can be applied. Furthermore, an exposure of longer than 1 second can be applied. If necessary, the spectral composition of light for use in the process of exposure can be controlled by means of a color filter. In the process of exposure, laser light can be used. Furthermore, the light-sensitive material of the invention may be exposed to light emitted from fluorescent substances excited by electron rays, X-rays, γ-rays, α-rays, and so forth.

A color-forming coupler, i.e., a compound capable of forming color through oxidative coupling with aromatic primary amine developers (e.g., phenylenediamine derivatives and aminophenol derivatives) in the color developing processing may be used in combination with polymer coupler latexes in the photographic emulsion layer of the light-sensitive material of the invention, or may be used singly in a layer where a polymer coupler latex is not used.

Typical examples of magenta couplers which can be used include a 5-pyrazolone coupler, a pyrazolobenzimidazole coupler, a cyanoacetylcoumarone coupler, and a closed acylacetonitrile coupler; typical examples of yellow couplers which can be used include an acylacetamide coupler (e.g., benzoylacetanilines and pivaloylacetanilides); and typical examples of cyan couplers which can be used include a naphthol coupler and a phenol coupler. These couplers are preferably non-diffusing ones containing a hydrophobic group called a ballast group in the molecule thereof. The couplers may be

either 4-equivalent or 2-equivalent relative to silver ion. Colored couplers having the effect of color correction or so-called DIR couplers releasing a development inhibitor as development proceeds can also be used. In addition to these DIR couplers, colorless DIR coupling compounds producing a colorless coupling reaction product and releasing a development inhibitor can be used.

Typical examples of the magenta couplers are described in, for example, U.S. Patents 2,600,788, 2,983,608, 3,062,653, 3,127,269, 3,311,476, 3,419,391, 3,519,429, 3,558,319, 3,582,322, 3,615,506, 3,834,908, 3,891,445, West German Patent 1,810,464, West German Patent Application (OLS) Nos. 2,408,665, 2,417,945, 2,418,959, 2,424,467, Japanese Patent Publication No. 6031/65, Japanese Patent Application (OPI) Nos. 20826/76, 58922/77, 129538/74, 74027/74, 159336/75, 42121/77, 74028/74, 60233/75, 26541/76, and 55122/78.

Typical examples of the yellow couplers are described in, for example, U.S. Patents 2,875,057, 3,265,506, 3,408,194, 3,551,155, 3,582,322, 3,725,072, 3,891,445, West German Patent 1,547,868, West German Patent Application Laid-Open Nos. 2,219,917, 2,261,361, 2,414,006, British Patent 1,425,020, Japanese Patent Publication No. 10783/76, Japanese Patent Application

(OPI) Nos. 26133/72, 73147/73, 102636/76, 6341/75, 123342/75, 130442/75, 21827/76, 87650/75, 82424/77 and 115219/77.

Typical examples of the cyan couplers are described in, for example, U.S. Patents 2,369,929, 2,434,272, 2,474,293, 2,521,908, 2,895,826, 3,034,892, 3,311,476, 3,458,315, 3,476,563, 3,583,971, 3,591,383, 3,767,411, 4,004,929, West German Patent Application (OLS) Nos. 2,414,830, 2,454,329, Japanese Patent Application (OPI) Nos. 59838/73, 26034/76, 5055/73, 146828/76, 69624/77 and 90932/77.

Typical examples of the colored couplers are described in, for example, U.S. Patents 3,476,560, 2,521,908, 3,034,892, Japanese Patent Publication Nos. 2016/69, 22335/63, 11304/67, 32461/69, Japanese Patent Application (OPI) Nos. 26034/76, 42121/77, and West German Patent Application (OLS) No. 2,418,959.

Typical examples of the DIR couplers are described in, for example, U.S. Patents 3,227,554, 3,617,291, 3,701,783, 3,790,384, 3,632,345, West German Patent Application (OLS) Nos. 2,414,006, 2,454,301, 2,454,329, British Patent 953,454, Japanese Patent Application (OPI) Nos. 69624/77, 12335/74, and Japanese Patent Publication No. 16141/76.

In addition to DIR couplers, compounds capable of releasing a development inhibitor with development may be incorporated in the light-sensitive material. Compounds which can be used include the ones described in, for example, U.S. Patents 3,297,445, 3,379,529, West German Patent Application (OLS) No. 2,417,914, Japanese Patent Application (OPI) Nos. 15271/77 and 9116/78.

The photographic light-sensitive material of the invention may contain inorganic or organic hardeners in the photographic emulsion layer and other hydrophilic colloid layers thereof. For example, chromium salts (e.g., chromium alum and chromium acetate), aldehydes (e.g., formaldehyde, glyoxal and glutaraldehyde), N-methylol compounds (e.g., dimethylourea and methylol-dimethylhydantoin), dioxane derivatives (e.g., 2,3-dihydroxydioxane), active vinyl compounds (e.g., 1,3,5-triacryloyl-hexahydro-s-triazine and 1,3-vinylsulfonyl-2-propanol), active halogen compounds (e.g., 2,4-dichloro-6-hydroxy-s-triazine), and mucohalogenic acids (e.g., mucochloric acid and mucophenoxychloric acid) can be used singly or in combination with each other.

In the light-sensitive material of the invention, if dyes and ultraviolet absorbers, for example, are contained in the hydrophilic colloid layer, they may be mordanted with cationic polymers, for example. For

example, the polymers described in British Patent 685,475, U.S. Patents 2,675,316, 2,839,401, 2,882,156, 3,048,487, 3,184,309, 3,445,231, West German Patent Application (OLS) No. 1,914,362, Japanese Patent Application (OPI) Nos. 47624/75 and 71332/75 can be used.

The light-sensitive material of the invention may contain, as anti-color foggants, hydroquinone derivatives, aminophenol derivatives, gallic acid derivatives, and ascorbic acid derivatives.

The light-sensitive material of the invention may contain ultraviolet absorbers in the hydrophilic colloid layer thereof. For example, aryl group-substituted benzotriazole, 4-thiazolidone, benzophenone, cinnamate, butadiene, and benzoxazole compounds, and further ultraviolet ray-absorbing polymers can be used. These ultraviolet absorbers may be fixed in the hydrophilic colloid layer.

Typical examples of the ultraviolet absorbers are described in, for example, U.S. Patents 3,533,794, 3,314,794, 3,352,681, Japanese Patent Application (OPI) No. 2784/71, U.S. Patents 3,705,805, 3,707,375, 4,045,229, 3,700,455, 3,499,762, and West German Patent Application Laid-Open No. 1,547,863.

The light-sensitive material of the invention may contain water-soluble dyes in the hydrophilic colloid layer thereof as filter dyes or for various purposes of,

e.g., preventing irradiation. Such dyes include oxonol dyes, hemioxonol dyes, styryl dyes, merocyanine dyes, cyanine dyes and azo dyes. Of these compounds, oxonol dyes, hemioxonol dyes and merocyanine dyes are useful.

In the light-sensitive material of the invention, the known anti-fading agents as described hereinafter can be used in combination. Color image stabilizers as used herein can be used singly or in combination with each other. The known anti-fading agents include hydroquinone derivatives, gallic acid derivatives, p-alkoxyphenols, p-oxyphenol derivatives, and bisphenols.

Typical examples of hydroquinone derivatives are described in U.S. Patents 2,360,290, 2,418,613, 2,675,314, 2,701,197, 2,704,713, 2,728,659, 2,732,300, 2,735,765, 2,710,801, 2,816,028 and British Patent 1,363,921. Typical examples of gallic acid derivatives are described in, for example, U.S. Patents 3,457,079 and 3,069,262. Typical examples of p-alkoxyphenols are described in, for example, U.S. Patents 2,735,765, 3,698,909, Japanese Patent Publication Nos. 20977/74 and 6623/77. Typical examples of p-oxyphenol derivatives are described in, for example, U.S. Patents 3,432,300, 3,573,050, 3,574,627, 3,764,337, Japanese Patent Application (OPI) Nos. 35633/77, 147434/77 and 152225/77. Typical examples of bisphenols are described in, for example, U.S. Patent 3,700,455.

The present invention is described in greater detail with reference to the following Example. However, the scope of the invention is not limited to this example.

EXAMPLE

A multilayer color photographic light-sensitive material was prepared by providing the layers as described below on a cellulose triacetate film support.

First Layer: Antihalation Layer

Gelatin layer containing black colloid silver

Second Layer: Intermediate Layer

Gelatin layer containing a dispersion of 2,5-di-tert-octylhydroquinone

Third Layer: Red-Sensitive Low Sensitivity Emulsion Layer

Silver iodobromide emulsion (silver iodide: 5 mol%, mean grain size: 0.5 μ)

| | |
|---|---|
| | 1.79 g/$m^2$ (amount of silver coated) |
| Sensitizing Dye I | $6\times10^{-5}$ mol per mol of silver |
| Sensitizing Dye II | $1.5\times10^{-5}$ mol per mol of silver |
| Coupler A | 0.06 mol per mol of silver |
| Coupler C | 0.003 mol per mol of silver |
| Coupler D | 0.003 mol per mol of silver |

Fourth Layer: Red-Sensitive High Sensitivity Emulsion Layer

Silver iodobromide emulsion (silver iodide: 4 mol%, mean grain size: 0.6 μ)

| | |
|---|---|
| | 1.2 $g/m^2$ (amount of silver coated) |
| Sensitizing Dye I | $3\times10^{-5}$ mol per mol of silver |
| Sensitizing Dye II | $1.2\times10^{-5}$ mol per mol of silver |
| Coupler A | 0.002 mol per mol of silver |
| Coupler F | 0.011 mol per mol of silver |
| Coupler C | 0.0016 mol per mol of silver |
| Compound A | 0.001 mol per mol of silver |

Amount of tricresyl phosphate coated

0.2 $m\ell/m^2$

Fifth Layer: Intermediate Layer

Same as the Second Layer.

Sixth Layer: Green-Sensitive Low Sensitivity Emulsion Layer

Silver iodobromide emulsion (silver iodide: 4 mol%, mean grain size: 0.5 μ)

| | |
|---|---|
| | 1.0 $g/m^2$ (amount of silver coated) |
| Sensitizing Dye III | $3\times10^{-5}$ mol per mol of silver |
| Sensitizing Dye IV | $1\times10^{-5}$ mol per mol of silver |
| Coupler B | 0.08 mol per mol of silver |

Coupler M 0.008 mol per mol of silver

Coupler D 0.0015 mol per mol of silver

Amount of tricresyl phosphate coated

0.3 mℓ/$m^2$

Seventh Layer: Green-Sensitive High Sensitivity Emulsion Layer

Silver iodobromide emulsion (silver iodide: 5 mol%, mean grain size: 0.75 μ)

1.6 g/$m^2$ (amount of silver coated)

Sensitizing Dye III $2.5 \times 10^{-5}$ mol per mol of silver

Sensitizing Dye IV $0.8 \times 10^{-5}$ mol per mol of silver

Coupler E 0.01 mol per mol of silver

Coupler M 0.003 mol per mol of silver

Coupler G 0.01 mol per mol of silver

Amount of tricresyl phosphate coated

0.8 mℓ/$m^2$

Eighth Layer: Yellow Filter Layer

Gelatin layer containing yellow colloid silver and a dispersion of 2,5-di-tert-octylhydroquinone in an aqueous solution of gelatin.

Ninth Layer: Blue-Sensitive Low Sensitivity Emulsion Layer

Silver iodobromide emulsion (silver iodide: 6 mol%, mean grain size: 0.7 μ)

0.5 g/$m^2$ (amount of silver coated)

Coupler Y 0.125 mol per mol of silver

Amount of tricresyl phosphate coated

0.3 mℓ/$m^2$

Tenth Layer: Blue-Sensitive High Sensitivity Emulsion Layer

Silver iodobromide emulsion (silver iodide: 6 mol%, mean grain size: 0.8 μ)

0.6 g/$m^2$ (amount of silver coated)

Coupler Y 0.04 mol per mol of silver

Amount of tricresyl phosphate coated

0.1 mℓ/$m^2$

Eleventh Layer: Protective Layer

Gelatin layer containing trimethyl methacrylate grains (mean grain size: about 1.5 μ)

The coupler or couplers for each layer were added to a solution of tricresyl phosphate and ethyl acetate and, after addition of sodium p-dodecylbenzenesulfonate as an emulsifier, dissolved therein by heating. The resulting solution was mixed with a 10% gelatin solution which had been heated and emulsified in a colloid mill.

To each layer, as well as the ingredients as described below, a gelatin hardener and a surfactant were added.

This light-sensitive material is designated as Sample 101.

The compounds as used in the preparation of the light-sensitive material were as follows:

Sensitizing Dye I: Anhydro-5,5'-dichloro-3,3'-di(γ-sulfopropyl)-9-ethyl-thiacarbocyanine hydroxide pyridinium salt

Sensitizing Dye II: Anhydro-9-ethyl-3,3'-di(γ-sulfopropyl)-4,5,4',5'-dibenzothiacarbocyanine hydroxide triethylamine salt

Sensitizing Dye III: Anhydro-9-ethyl-5,5'-dichloro-3,3'-di(γ-sulfopropyl)oxacarbocyanine sodium salt

Sensitizing Dye IV: Anhydro-5,6,5',6'-tetrachloro-1,1'-diethyl-3,3'-di{β-[β-(γ-sulfopropoxy)ethoxy]ethylimidazolocarbocyanine}hydroxide sodium salt

Sensitizing Dye V: Anhydro-6,6'-dichloro-5'-cyano-1,1'-diethyl-5-trifluoromethyl-3-(p-sulfophenethyl)-3'-(4-sulfobutyl)benzimidazolocarbocyanine hydroxide potassium salt

Coupler A

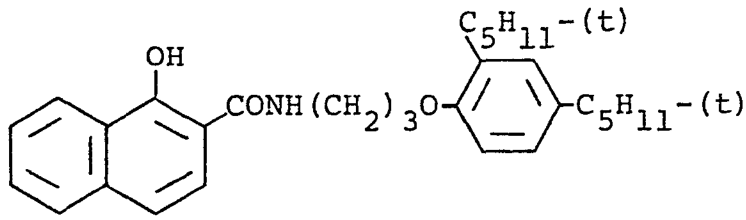

Coupler B

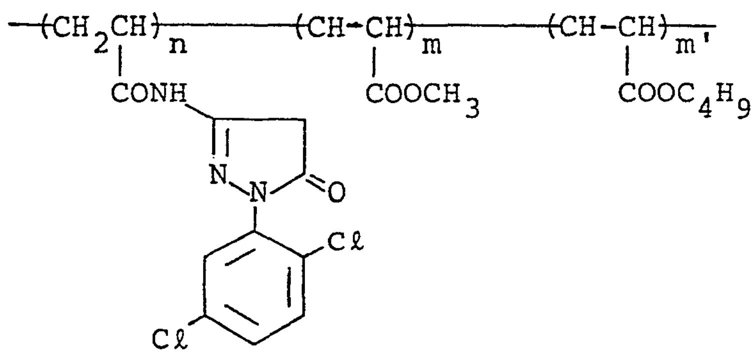

n/(m+m')=1. m/m'=1:1 (by weight)

molecular weight: about 40,000

Coupler C

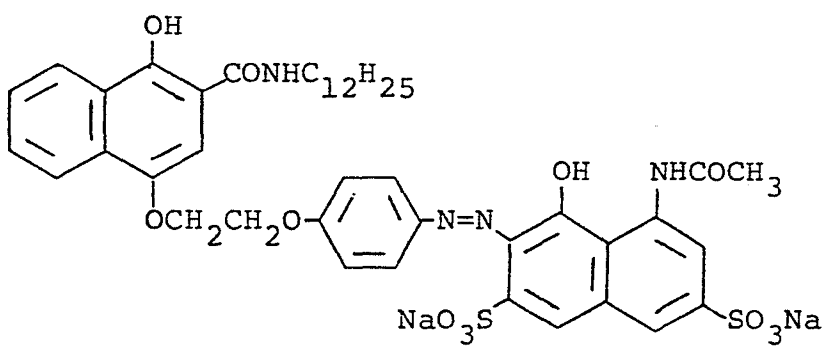

Coupler D

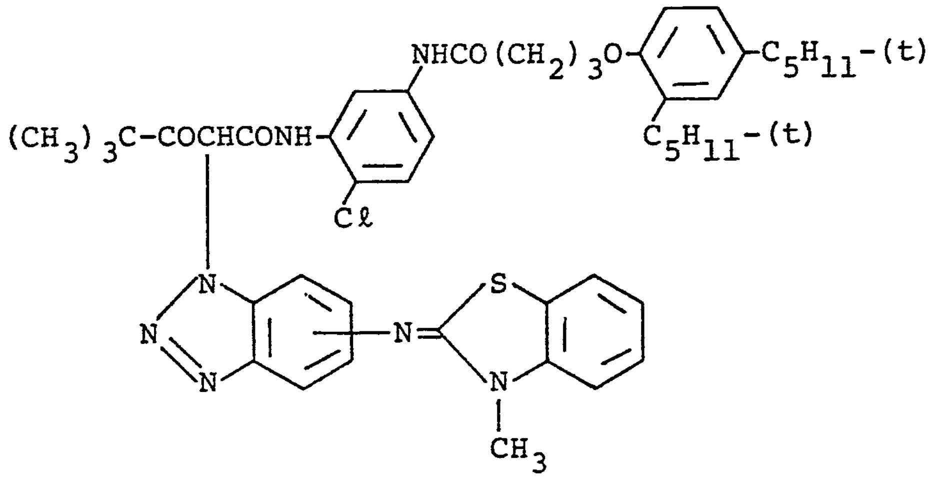

Coupler E

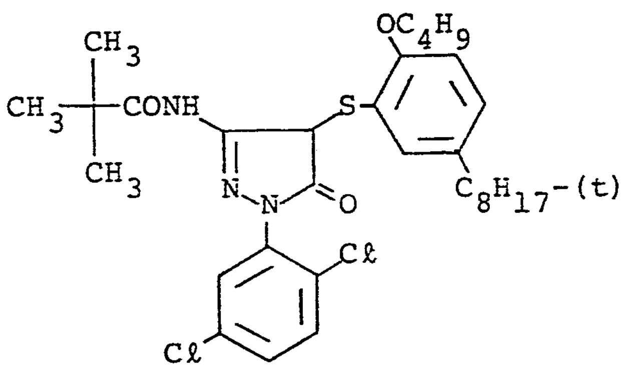

Coupler F

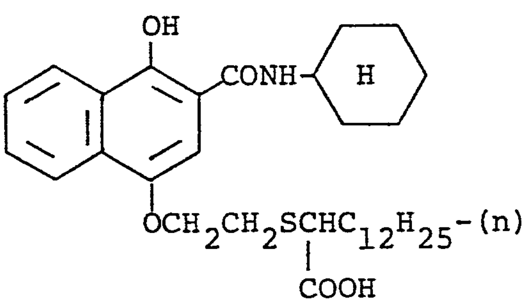

Coupler G

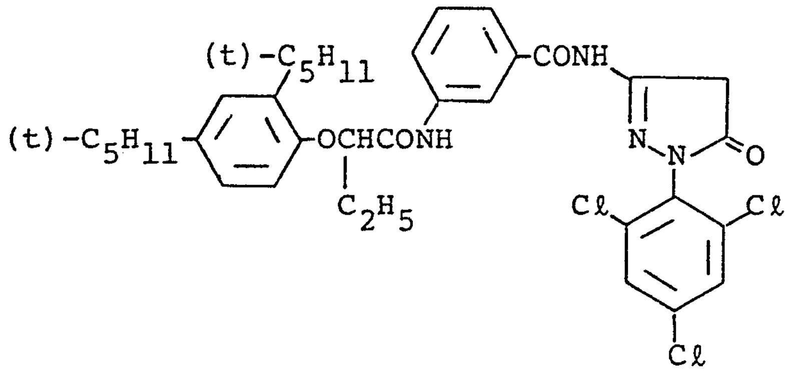

Coupler H

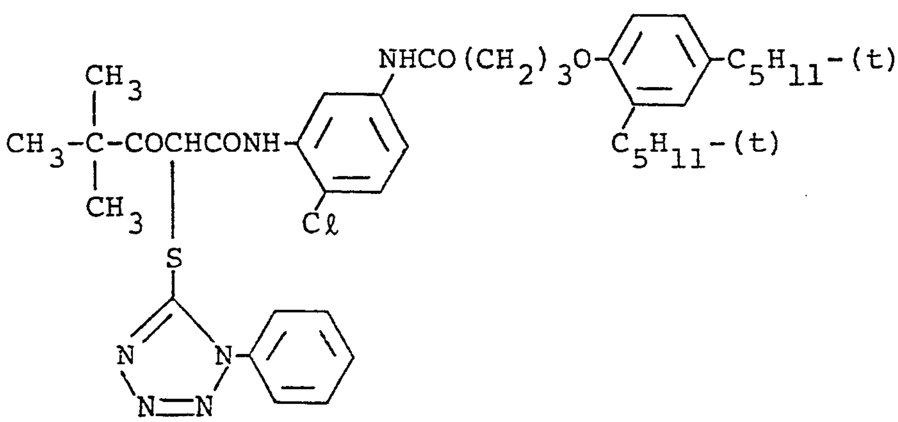

Compound A

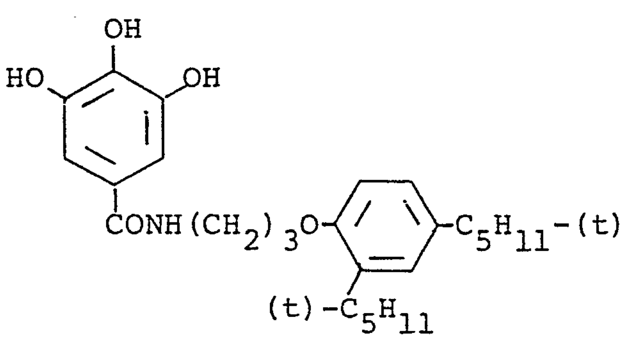

Coupler I

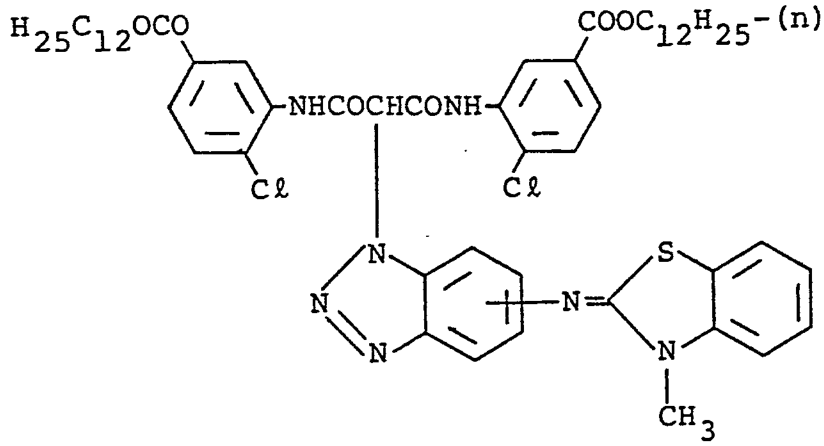

Coupler M

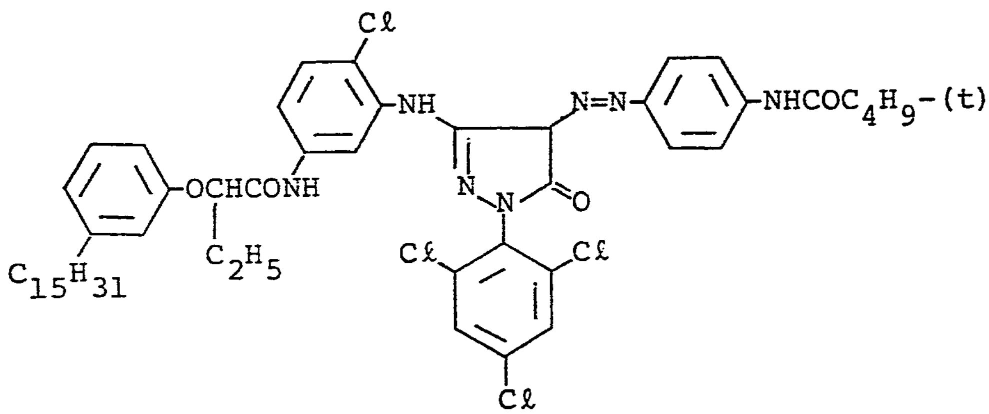

Coupler Y

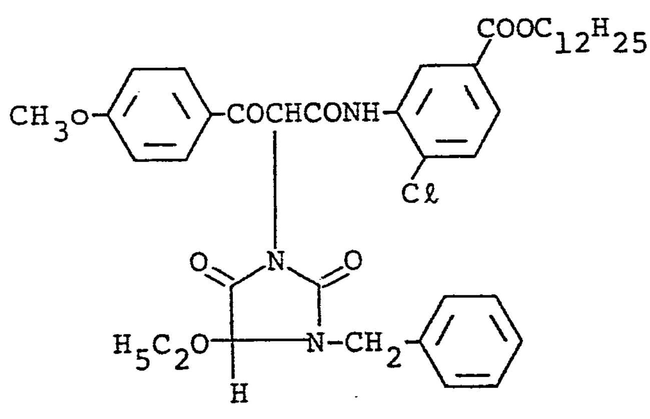

A series of light-sensitive materials, Samples 102 to 107, were prepared in the same manner as in the preparation of Sample 101 except that the type and amount of Coupler D in the Sixth Layer were changed as shown in Table 1. In addition, light-sensitive materials, Samples 108 and 109, were prepared in the same manner as in the preparation of Samples 101 and 103 except that Sensitizing Dye IV of the Sixth and Seventh Layers was replaced by Sensitizing Dye V.

Each light-sensitive material was exposed to white light through a pattern for determination of MTF and then was subjected to the developing processing as described below. The light-sensitive material was also exposed wedgewise to white light, and its sensitivity and gradation were determined.

The developing processing was performed at 38°C as follows:

| | | |
|---|---|---|
| 1. | Color Development | 3.25 minutes |
| 2. | Bleaching | 6.5 minutes |
| 3. | Rinsing | 3.25 minutes |
| 4. | Fixing | 6.5 minutes |
| 5. | Rinsing | 3.25 minutes |
| 6. | Stabilization | 3.25 minutes |

The composition of the processing solution as used at each step was as follows:

Color Developer

| | |
|---|---|
| Sodium Nitrilotriacetate | 1.0 g |
| Sodium Sulfite | 4.0 g |
| Sodium Carbonate | 30.0 g |
| Potassium Bromide | 1.4 g |
| Hydroxylamine Sulfate | 2.4 g |
| 4-(N-Ethyl-N-β-hydroxyethylamino)-2-methylaniline Sulfate | 4.5 g |
| Water to make | 1 liter |

Bleaching Solution

| | |
|---|---|
| Ammonium Bromide | 160.0 g |
| Ammonia Water (28%) | 25.0 mℓ |
| Sodium Iron Ethylenediaminetetraacetate | 130 g |
| Glacial Acetic Acid | 14 mℓ |
| Water to make | 1 liter |

Fixer

| | |
|---|---|
| Sodium Tetrapolyphosphate | 2.0 g |
| Sodium Sulfite | 4.0 g |
| Ammonium Thiosulfate (70%) | 175.0 mℓ |
| Sodium Hydrogensulfite | 4.6 g |
| Water to make | 1 liter |

Stabilizing Solution

| | |
|---|---|
| Formalin | 8.0 mℓ |
| Water to make | 1 liter |

For the thus-processed materials, MTF of the magenta color image was measured, and MTF at a space frequency of 5 cycle/mm is shown in Table 2. Also, the density of the cyan color image at a point where the exposure amount was larger by 0.5 (as a logarithmic value) than that at a fog density of +0.2 was measured, and the results are also shown in Table 2. This point is referred to as a point of log E=0.5.

It can be seen that if the DIR couplers of the invention are used, the MTF of the green-sensitive emulsion layer is greatly increased and the development inhibitor released diffuses even in the red-sensitive emulsion layer, as a result of which the color-formation of the red-sensitive emulsion layer is prevented; that is, the so-called interlayer effect is greatly increased. Thus, the DIR compounds of the invention greatly contribute to an increase in color reproductivity.

TABLE 2

| Sample No. | Coupler | Amount (molar ratio) | MTF Value at 5 Cycle/mm | Density of Cyan Image at Log E=0.5 |
|---|---|---|---|---|
| 101 (Comparative Example) | D | 1.0 | 1.05 | 1.70 |
| 102 ( " ) | II | 2.0 | 1.03 | 1.82 |
| 103 (Example of the Invention) | (1) | 2.2 | 1.21 | 1.51 |
| 104 ( " ) | (4) | 5.0 | 1.10 | 1.55 |
| 105 ( " ) | (5) | 1.0 | 1.23 | 1.52 |
| 106 ( " ) | (6) | 1.0 | 1.20 | 1.49 |
| 107 ( " ) | (9) | 3.0 | 1.25 | 1.47 |

The degrees of diffusion of Couplers D and H (comparative couplers) and of Couplers (1) and (4) (the couplers of the invention) are 0.3, 0.2, 0.70 and 0.44, respectively. Thus, it can be seen that as the degree of diffusion increases, the MTF value increases.

When the degree of diffusion is 0.44, the MTF value is 1.10 which is beyond an MTF value at which an improvement in sharpness can be observed with the eye. This clearly demonstrates the effect of the invention.

Samples 108 and 109 produced the same results as did Samples 101 and 103.

While the invention has been described in detail and with reference to specific embodiments thereof, it will be apparent to one skilled in the art that various changes and modifications can be made therein without departing from the spirit and scope thereof.

WHAT IS CLAIMED IS:

1. A color photographic silver halide light-sensitive material, comprising:

a support base having thereon:

a silver halide emulsion layer; and

a development inhibitor-releasing coupler capable of releasing an inhibitor having a degree of diffusion ranging between 0.4 and 0.95 on reacting with an oxidation product of a developing agent during development.

2. A material as claimed in Claim 1, wherein the coupler contains as a releasing group a naphtho-triazolyl group or a group selected from the group consisting of (I), (II) and (III):

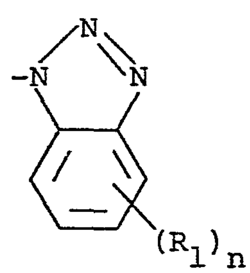

(I)

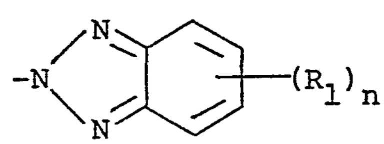

(II)

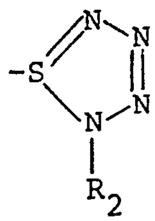

(III)

wherein n is 1 or 2; when n is 1, $R_1$ is a pentanamido group, a hexanamido group, a propoxy group, an N-butanecarbamoyl group, an N-pentylcarbamoyl gorup, an N-hexylcarbamoyl group, a butoxy group, a pentyloxy group, or an ethyl group, and when n is 2, $R_1$ is a methyl group; and $R_2$ is a propyl group or a substituted phenyl group (the substituent is selected from the class consisting of an N-methylsulfamoyl group, a ureido group, a carbamoyl group, an N-methylcarbamoyl group, a methanesulfonamido or acetamido group, and an alkoxyacylamino group.

3. A material as claimed in Claim 2, wherein $R_2$ is a substituted phenyl group substituted with an alkyl group containing 1 or 2 carbon atoms.

4. A material as claimed in Claim 2, wherein the development inhibitor-releasing couplers are selected from the group of compounds consisting of (IV), (V) and (VI):

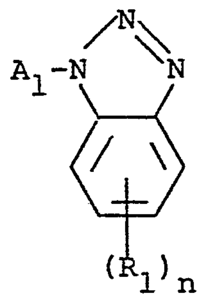

(IV)

or

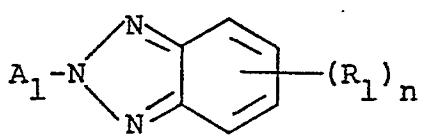

(IV)

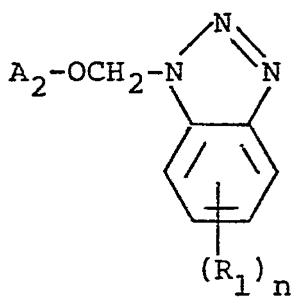

(V)

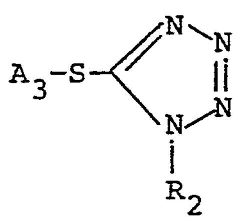

(VI)

wherein $A_1$ is a radical derived from a yellow coupler or a magenta coupler by removing one hydrogen atom positioned at the coupling position thereof; $A_2$ is a radical derived from a cyan coupler by removing one

hydrogen atom positioned at the coupling position thereof; $A_3$ is a radical derived from a yellow coupler, a magenta coupler or a cyan coupler by removing one hydrogen atom from the coupling position thereof; and $R_1$, $R_2$ and n are the same as in Claim 2.

5. A material as claimed in Claim 4, wherein the development inhibitor-releasing coupler is present in an amount in the range of 0.00001 to 0.5 mol per mol of silver.

6. A material as claimed in Claim 5, wherein the development inhibitor-releasing coupler is present in an amount in the range of 0.001 to 0.05 mol per mol of silver.

Fig. 1

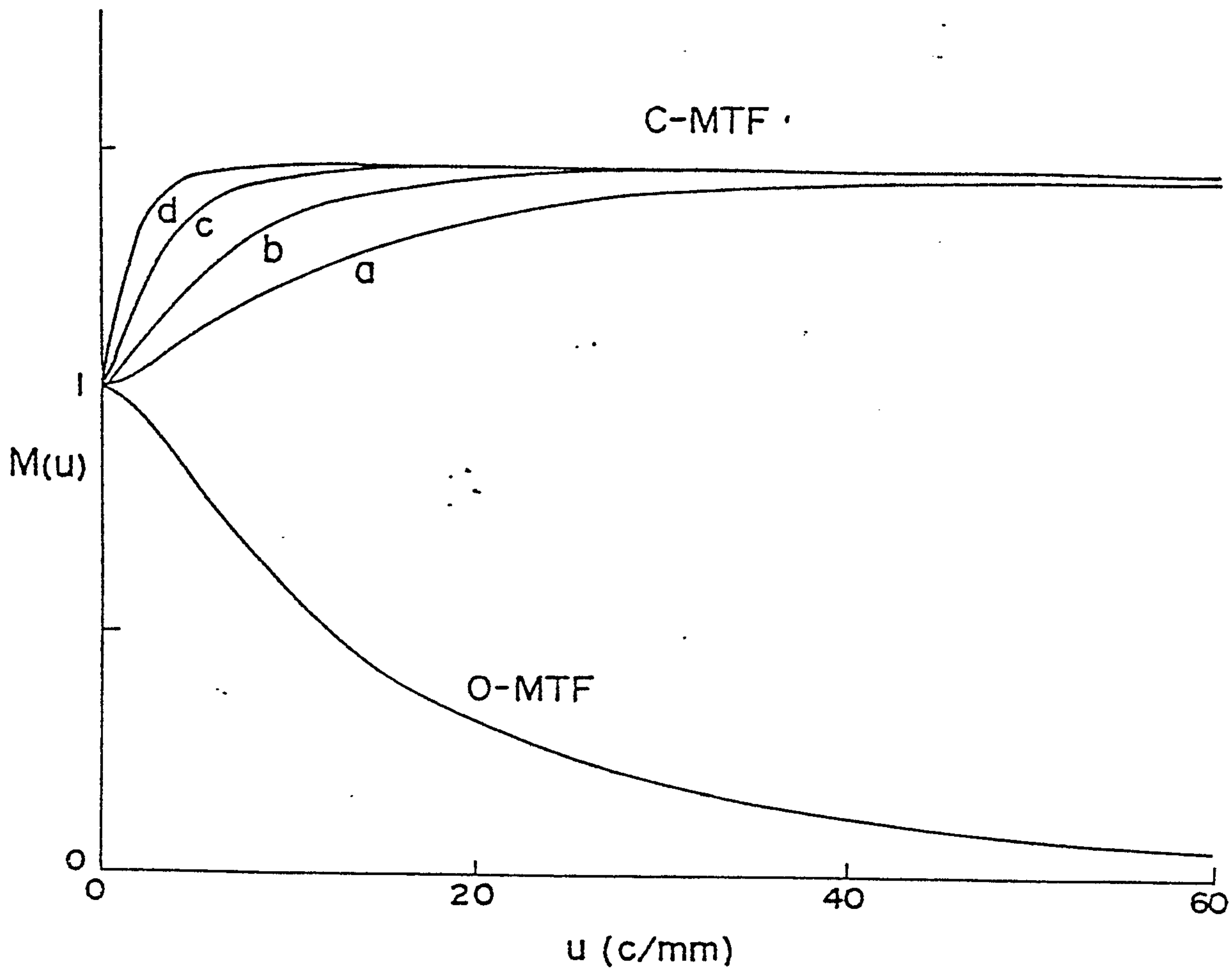

Fig. 2